Europäisches Patentamt

**European Patent Office**

**Office européen des brevets**

(19)

(11) Publication number: **0 004 710**
**B1**

(12) **EUROPEAN PATENT SPECIFICATION**

(45) Date of publication of patent specification: **04.11.81**

(21) Application number: **79300356.7**

(22) Date of filing: **08.03.79**

(51) Int. Cl.³: **C 07 C 49/80,**
**C 07 C 45/28, C 07 C 45/00**
**//C07C113/04, C07C25/18,**
**C07C17/22**

(54) Preparation of 4,4'-difluorobenzophenone.

(30) Priority: 31.03.78 GB 1266478
10.08.78 GB 3284278
25.08.78 GB 3466278

(43) Date of publication of application:
17.10.79 Bulletin 79/21

(45) Publication of the grant of the European patent:
04.11.81 Bulletin 81/44

(84) Designated Contracting States:
BE CH DE FR GB IT NL SE

(56) References cited:

BULLETIN DE LA SOCIETE CHIMIQUE DE
FRANCE, 1951,
PARIS
J. LICHTENBERGER et al.: "Préparation
de quelques dérivés fluorés en série aromatique",
pages 318—325

(73) Proprietor: IMPERIAL CHEMICAL INDUSTRIES
LIMITED
Imperial Chemical House Millbank
London SW1P 3JF (GB)

(72) Inventor: Staniland, Philip Anthony
1 Desborough Drive
Tewin Wood Herts (GB)
Inventor: Bowden, Roy Dennis
5 Red Lane
Frodsham Cheshire (GB)
Inventor: Burgess, Leslie
1 Poolside Road
Runcorn Cheshire (GB)
Inventor: Clark, Raymond John
8 Ramilies Road
Mossley Hill Liverpool (GB)

(74) Representative: Roberts, John Stanley et al,
IMPERIAL CHEMICAL INDUSTRIES LIMITED
Legal Department: Patents Thames House North
Millbank
London SW1P 4QG (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Preparation of 4,4'-difluorobenzophenone

The present invention relates to a process for the preparation of 4,4'-diflourobenzophenone.

4,4'-diflourobenzophenone is a useful monomeric reactant for the production of aromatic polyketone polymers whole molecular chains comprise phenylene groups, oxygen atoms and ketone groups. For example, 4,4'-diflourobenzophenone may be condensed with the di(alkali metal) salts of numerous aromatic bisphenols to form different polyketones as described in British Patents 1 078 234 and 1 414 421. Alternatively 4,4'-difluorobenzophenone may be condensed with aromatic bisphenols in the presence of alkali metal carbonates or bicarbonates to form polyketones, as in the process described in Canadian Patent 847 963.

Known methods for the production of 4,4'-difluorobenzophenone involve the use of Friedel Crafts acylations employing fluorobenzene as starting material. Such methods include reacting fluorobenzene with p-fluoro-benzoyl chloride or phosgene. However fluorobenzene is an expensive material and the reaction of fluorobenzene with phosgene produces an isomeric mixture which requires costly purification. Furthermore the handling and disposal of large amounts of spent catalysts (as would be required on a production scale) would also be costly.

In Bulletin de la Societe Chimique de France, 1951, page 322, there is described a method for the preparation of 4,4'-diflouro-diphenylmethane from 4,4' - diamino - diphenylmethane via the corresponding bis-(diazonium fluoborate); the 4,4' - difluoro - diphenylmethane thus produced is separated, dissolved in carbon disulphide and oxidised to 4,4'-difluorobenzophenone by means of chromyl chloride.

We have now found that 4,4'-diflourobenzophenone may be prepared by a process comprising, as first step, diazotization of 4,4'-diamino-diphenylmethane in solution in anhydrous or concentrated aqueous hydrogen fluoride; surprisingly, when the decomposition of the diazonium fluoride is carried out in the said solution in the presence of nitrous acid or nitrite ions, oxidation of the methylene group to the carbonyl group of the desired product can be achieved directly, without the need for a subsequent separate oxidation stage. The reaction mechanism is not fully understood; when the decomposition of the diazonium fluoride is carried out in the presence of nitrous acid or nitrite ions 4,4'-difluoro-diphenylmethane is not necessarily formed as an intermediate (i.e. fluorination does not necessarily precede oxidation of the methylene group).

Thus according to the present invention there is provided a process for the preparation of the 4,4' - difluoro - benzophenone from 4,4' - diamino - diphenylmethane via a corresponding diazonium fluoride, characterised in that 4,4'-diamino-diphenylmethane is diazotised in solution in anhydrous or concentrated aqueous hydrogen fluoride and that the diazonium fluoride thus produced is thermally decomposed in the presence of nitrous acid or nitrite ions as oxidising agent, the total quantity of nitrous acid or nitrite ions employed in the overall process being at least 4 moles per mole of 4,4'-diamino-diphenylmethane.

The nature of the ionic species present in anhydrous or concentrated aqueous hydrogen fluoride is not fully understood and the terms "nitrous acid" and "nitrite", as used herein, include species derived from nitrous acid or nitrite ions in the reaction medium, for example HF complexes of $N_2O_3$.

The process is most conveniently carried out by introducing nitrite ions in the form of an alkali metal nitrite, preferably sodium nitrite. The nitrite is preferably introduced in the form of a solution in anhydrous or concentrated aqueous hydrogen fluoride.

In general, the yield of the desired product decreases as the proportion of water in the reaction mixture increases. If desired, anhydrous hydrogen fluoride may be used as the initial medium and as the solvent for the nitrous acid or nitrite introduced. It will be appreciated, however, that water is produced in the overall process leading from 4,4'-diamino-diphenylmethane to 4,4'-difluoro-benzophenone.

Accordingly, there is no point in rigorous exclusion of water from the reaction medium but if aqueous hydrogen fluoride is employed as the initial medium this preferably contains at least 60%, preferably at least 70%, by weight of HF. The proportion of water initially present is preferably chosen so that at the end of the process the proportion of water (including that produced in the process) is less than 50% by weight of the total of HF and water; preferably the final proportion of water is less than 40%, particularly less than 30%, on this basis.

From stoichiometric considerations, there should be at least 4 moles of HF present per mole of 4,4'-diamino-diphenylmethane. However since HF is also employed as solvent it is usually present in a large excess over stoichiometric, e.g. in a 5—30 fold excess over the stoichiometrically necessary quantity. A large amount of HF will also minimise (by dilution) the effect of the water produced in the diazotization reaction.

The overall process may be carried out in a variety of ways. If desired, the diazotization and the decomposition/oxidation may be combined into a single stage by carrying out the diazotization at a temperature of at least 40°C, using at least 4 moles, for example from 4 to 6 moles, of nitrous acid or nitrite per mole of 4,4'-diamino-diphenylmethane. A greater excess may be used if desired by in general no benefit is secured by increasing the proportion of nitrous acid or nitrite beyond this range.

Thus the nitrous acid or nitrite may be added continuously or incrementally to a solution of 4,4'-diamino-diphenylmethane in anhydrous or concentrated aqueous hydrogen fluoride maintained at a temperature of at least 40°C, the total proportion of nitrous acid and/or nitrite introduced being at least 4 moles per mole of 4,4'-diamino-diphenylmethane; the temperature of the reaction mixture may then be maintained at 40°C or above for such further period as may be necessary to effect conversion to 4,4'-difluorobenzophenone. In this embodiment of the invention the temperature of the reaction mixture is preferably maintained at 50°C or above (for example from 50°C to 100°C, especially from 50°C to 70°C) both during the addition of the nitrous acid or nitrite and during any further period of heating.

Alternatively, the diazotization may be followed by decomposition/oxidation as a separate stage. Thus the process of diazotization may be carried out at a temperature of 5°C or below, the reaction mixture subsequently being heated to a temperature of at least 40°C in the presence of excess nitrous acid or nitrite ions. In this embodiment, the proportion of nitrous acid or nitrite introduced at the relatively low temperature may simply be the stoichiometric proportion required for diazotization (2 moles of nitrous acid or nitrite per mole of 4,4'-diaminodiphenylmethane) or a slight excess over this proportion (for example a 5% excess); the balance of the nitrous acid or nitrite required to provide the total of at least 4 moles per mole may then be introduced when the temperature of the reaction mixture has subsequently been raised to 40°C or above. If desired, however, all or part of this balance of nitrous acid or nitrite required may be introduced at the relatively low temperature. In this embodiment also, the temperature of the reaction mixture is preferably maintained at 50°C or above during the heating stage and the total proportion of nitrous acid and/or nitrite introduced is at least 4 moles per mole of 4,4'-diamino-diphenylmethane (for example from 4 to 6 moles per mole).

The time of heating required will depend upon such factors as the concentration of hydrogen fluoride, the excess of nitrous acid or nitrite and the temperature employed. In general the temperature of the reaction mixture will be maintained at 40°C or above for a period ranging from about half an hour to several hours after addition of the nitrous acid or ntirite has been completed.

The heating is conveniently carried out under reflux conditions. The proportions of water at this stage may be increased, if necessary, to allow the required temperature to be maintained under reflux conditions; alternatively, superatmospheric pressure may be used.

After completion of the reaction, 4,4'-difluorobenzophenone may be separated from the reaction mixture by conventional methods, for example solvent extraction.

The invention is illustrated by the following Examples.

## Example 1

A solution of 4,4'-diamino-diphenylmethane (79.2g, 0.4 mole) in aqueous 85% w/w hydrogen flouride (300g) was prepared in a reactor equipped with a stirrer and condenser.

A solution of sodium nitrite (112.6g, 1.63 mole) in aqueous 85% w/w hydrogen fluoride (700g) was prepared at −5°C. The cold nitrite solution was continuously added dropwise over 15 minutes to the solution of 4,4'-diaminodiphenylamine which was initially at −16°C. The reaction mixture was cooled so that the temperature did not rise above 2°C during addition of the nitrite solution.

After addition of the nitrite solution had been completed, the mixture was heated to reflux temperature (55°C to 60°C), maintained under reflux for $2\frac{1}{4}$ hours and then allowed to cool to room temperature. The reaction mixture was subjected to solvent extraction with diethyl ether, the extract washed with water and the ether removed. The product was recrystallised from an 80/20 weight/weight mixture of industrial methylated spirits and water to yield 35.7g (41%) of 4,4'-difluoro-benzophenone, melting point 108°C. The structure of the product was confirmed by infra-red and nuclear magnetic resonance spectroscopy.

## Example 2

The general procedure was similar to that described in Example 1.

A solution of sodium nitrite (155g, 2.24 mole) in aqueous 95% w/w hydrogen fluoride (575 ml) was prepared at −10°C. A portion of this solution (210 ml, 0.815 mole of nitrite) was added rapidly, with stirring, to a solution of 4,4'-diamino-diphenylmethane (79.2g, 0.40 mole) in aqueous 95% w/w hydrogen fluoride (150 ml), the temperature being maintained between −20°C and −10°C. This addition effected diazotization; the rest of the nitrite solution was added over a period of 2 hours while the temperature was maintained at −10°C or below. The mixture was then heated to reflux (55°C to 57°C) over a period of 45 minutes and maintained under reflux for 3 hours.

After cooling to room temperature, the reaction mixture was extracted with three 200ml portions of carbon tetrachloride. The combined extracts were washed with water and the solvent was removed by distillation. The product was recrystallized from an 80/20 w/w mixture of industrial methylated spirits/water to yield 68.5g (78%) of 4,4'-difluorobenzophenone (melting point 108°C).

### Example 3

The general procedure was similar to that described in Example 1.

Solid sodium nitrite (127g, 1.84 mole) was added slowly, with stirring, to a solution of 4,4'-diamino-diphenylmethane (79.2g, 0.40 mole) in aqueous 90% w/w hydrogen fluoride (590g), the temperature being maintained between −20°C and −10°C. The mixture was then heated to reflux (62°C to 64°C) over a period of 1.5 hours and maintained under reflux for 1 hour.

After cooling to room temperature, the reaction mixture was extracted with three 200ml portions of carbon tetrachloride. The product was extracted and recrystallised (as described in Example 2) to yield 53.5g (61%) of 4,4'-difluorobenzophenone (melting point 108°C).

### Claims

1. A process for the preparation of 4,4'-difluorobenzophenone from 4,4'-diamino-diphenylmethane via a corresponding diazonium fluoride, characterized in that 4,4'-diamino-diphenylmethane is diazotized in solution in anhydrous or concentrated aqueous hydrogen fluoride and that the diazonium fluoride thus produced is thermally decomposed, in the presence of nitrous acid or nitrite ions as oxidising agent, to yield 4,4'-difluorobenzophenone, the total quantity of nitrous acid or nitrite ions employed in the overall process being at least 4 moles per mole of 4,4'-diamino-diphenylmethane.

2. A process according to Claim 1, characterized by the addition of nitrous acid or nitrite ions continuously or incrementally over at least part of the total reaction period to a solution of 4,4'-diamino-diphenylmethane in anhydrous or concentrated aqueous hydrogen fluoride maintained at a temperature of at least 40°C.

3. A process according to Claim 2, characterized in that the solution in hydrogen fluoride is maintained at a temperature in the range 50°C to 70°C.

### Revendications

1. Procédé de préparation de la 4,4'-difluoro-benzophénone à partir du 4,4'-diamino-diphénylméthane par l'intermédiaire d'un fluorure de diazonium correspondant, caractérisé en ce qu'on diazote le 4,4'-diaminodiphénylméthane en solution dans le fluorure d'hydrogène anhydre ou aqueux concentré et on décompose thermiquement le fluorure de diazonium ainsi formé, en présence d'acide nitreux ou d'ions nitrite comme agent oxydant, pour obtenir la 4,4'-difluorobenzophénone, la quantité totale d'acide nitreux ou d'ions nitrite utilisée dans le procédé complet étant d'au moins 4 moles/mole de 4,4'-diaminodiphénylméthane.

2. Procédé suivant la revendication 1, caractérisé par l'addition de l'acide nitreux ou des ions nitrite de manière continue ou par incréments pendant au moins une partie de la durée de réaction totale à une solution de 4,4'-diaminodiphénylméthane dans le fluorure d'hydrogène anhydre ou aqueux concentré maintenue à une température d'au moins 40°C.

3. Procédé suivant la revendication 2, caractérisé en ce que la solution dans le fluorure d'hydrogène est maintenue à une température de 50 à 70°C.

### Patentansprüche

1. Verfahren zur Herstellung von 4,4'-Difluorbenzophenon aus 4,4'-Diamino-diphenylmethan über ein entsprechendes Diazoniumfluorid, dadurch gekennzeichnet, daß 4,4'-Diamino-diphenylmethan in Lösung in wasserfreiem oder konzentriertem wäßrigen Fluorwasserstoff diazotiert wird und daß das so hergestellte Diazoniumfluorid in Gegenwart von salpetriger Säure oder Nitritionen als Oxidationsmittel unter Bildung von 4,4'-Difluor-benzophenon thermisch zersetzt wird, wobei die Gesamtmenge der im gesamten Verfahren verwendeten salpetrigen Säure oder Nitritionen mindestens 4 Mol je Mol 4,4'-Diaminodiphenylmethan beträgt.

2. Verfahren nach Anspruch 1, gekennzeichnet durch die kontinuierliche oder portionsweise Zugabe von salpetriger Säure oder Nitritionen während mindestens eines Teils der gesamten Reaktionszeit zu einer Lösung von 4,4'-Diamino-diphenylmethan in wäßrigem oder konzentriertem wäßrigen Fluorwasserstoff, die auf eine Temperatur von mindestens 40°C gehalten wird.

3. Verfahren nach Anspruch 2, dadurch gekennzeichnet, daß die Lösung in Fluorwasserstoff auf eine Temperatur von 50 bis 70°C gehalten wird.